# EUROPEAN PATENT APPLICATION

(11) **EP 4 309 644 A1**
(43) Date of publication of application: **24.01.2024**
(21) Application number: 22461582.3
(22) Date of filing: 22.07.2022
(51) Int. Cl.: A61K 9/08, A61F 9/00, A61K 31/5377, A61K 31/5575, A61K 9/00, A61K 47/12, A61K 47/26, A61K 45/06, A61K 31/382, A61K 31/498, A61K 33/38, A61P 27/06

(54) **PRESERVATIVE-FREE OPHTHALMIC COMPOSITION COMPRISING AN ANTIGLAUCOMA AGENT**

(71) Applicant: Warszawskie Zaklady Farmaceutyczne Polfa S.A., 01-207 Warszawa (PL)
(72) Inventor: ROLA-LASEK, Marzena, 02-315 Warszawa (PL); ZARCZUK, Jakub, 02-315 Warszawa (PL); MALIK, Katarzyna, 02-315 Warszawa (PL)

(57) **Abstract**

The present invention relates to a multi-dose dispensing device filled with a preservative-free ophthalmic composition comprising a carbonic anhydrase inhibitor, characterized in that the composition remains microbiologically acceptable during use for a long period, of at least 90 days after the first opening of the container, and wherein said microbiological acceptability is assessed in a microbiological test comprising repeated microbial challenge with four microorganisms consisting of one gram-negative bacterium, one gram-positive bacterium, one fungus of the *Aspergillus* genus and *Candida albicans.*

## Description

### Technical field

The present invention relates to a preservative-free ophthalmic composition comprising an antiglaucoma agent as active ingredient, in particular, a carbonic anhydrase inhibitor, which is contained in a multi-dose dispensing device, and which remains microbiologically acceptable for a long period during use.

### State of the art

Glaucoma is a blinding disease characterized by the death of retinal ganglion cells and their axons and is a major cause of irreversible blindness worldwide. An elevated intraocular pressure (IOP) is the main risk factor for disease progression, and, therefore, lowering IOP is the standard strategy for glaucoma therapy.

Most common topical IOP-lowering therapies include carbonic anhydrase inhibitors (CAIs), alpha-adrenergic agonists, prostaglandin analogues, cholinergic agonists and beta-blockers.

Topical CAIs, in particular, include dorzolamide and brinzolamide. They were developed as an alternative to oral CAIs for treating glaucoma, to mitigate their systemic side effects through local administration, and were both approved in the mid-1990s for the management of open-angle glaucoma. CAIs reduce IOP by suppressing aqueous humour production at the ciliary bodies through the prevention of the interconversion of carbon dioxide and carbonic acid. Nowadays, topical CAIs still remain a widely used therapeutic option for glaucoma, sometimes as second-line agents when prostaglandin analogues and/or beta-blockers are contraindicated, and are frequently used as part of combination therapies, such as dorzolamide-timolol, brinzolamide-timolol or brinzolamide-brimonidine, for example (Stoner et al., Br. J. Ophthalmol. 2021, Aug 25:bjophthalmol-2021-319530. Epub ahead of print).

Brimonidine, for example, which is frequently used in combination with CAIs, is a selective alpha2-adrenergic receptor agonist, whose mechanism of action for IOP lowering is also related to a decrease in aqueous humour production in the ciliary body epithelium.

Timolol, also frequently used in combination with CAIs, is a beta-receptor antagonist, which reduces aqueous humour production in the ciliary body by decreasing intracellular cAMP concentration.

Long-term or even lifelong lasting therapy with IOP-lowering agents is generally required for glaucoma treatment. Therefore, for patient convenience, the ophthalmic solutions are usually available as multi-dose bottles, providing medication for several months in one single package. For ensuring sterility, in general, the inclusion of antimicrobial preservative in the solutions is required, frequently, the quaternary ammonium benzalkonium chloride (BAK).

However, there is evidence that BAK may cause or enhance harmful consequences on the ocular surface, including the tear film, cornea and conjunctiva, particularly after prolonged use, as is the case of anti-glaucoma medications (Baudouin et al., Preservatives in eyedrops: The good, the bad and the ugly, Prog. Retin. Eye Res., 2010, 29, 312-334).

Due to those well-documented BAK-related side effects, a need for preservative-free topical antiglaucoma medications has arisen and, consequently, several preservative-free ophthalmic dosage forms have been developed. For avoiding microbiological contamination, in the absence of preservatives, the safest and most common option so far in the art are single-dose units. However, some drawbacks have been reported for single-dose units: the higher cost, the possibility of corneal injury, and the perceived difficulty with their handling by elderly patients (Holló, et al., Preservative-Free Prostaglandin Analogs and Prostaglandin/Timolol Fixed Combinations in the Treatment of Glaucoma: Efficacy, Safety and Potential Advantages, Drugs, 2018, 78(1), 39-64).

Therefore, preservative-free multiple-dose (PFMD) dosage forms, in the form of bottles containing enough ophthalmic solution for several uses, are the optimal option for both avoiding the adverse effects of the preservative and also providing a more economical and more convenient form for the patients for the long-term treatment of glaucoma. However, avoiding microbial contamination in those unpreserved solutions is of the utmost importance, and it is not trivial, as the compositions are generally aqueous and, therefore, susceptible to microbial contamination, namely, once the bottle is opened and the composition remains in use during several days or weeks.

For avoiding microbiological contamination in PFMD systems, several closure devices have been developed, which, according to different strategies, set up a barrier to prevent bacteria from entering into the container. Different systems are available, for example, those based on pump systems (such as COMOD^{®} and 3K^{®} systems) or those based on the use of non-return valves combined with filters for the compensating air entering into the container (such as Novelia^{®} and OSD^{®}) (Yoon et al., Packaging Development: Multi-dose container closure for preservative free products, extractable/leachables from packaging, new technologies, in: Neervannan, S., Kompella, U.B. (eds) Ophthalmic Product Development. AAPS Advances in the Pharmaceutical Sciences Series, vol 37. Springer, Ophthalmic Product Development, 229-245).

Once the dosage form is opened, the sterility and microbiological integrity of the composition must be ensured during the whole period of use, irrespective of any possible challenging situation arisen during use, typically, the contact of the tip of the dispenser with contaminated surfaces. From a regulatory point of view, furthermore, the evidence of the required sterility in such "in use" conditions must be conclusive. That is why, in general, the PFMD systems available so far in the market for anti-glaucoma medications are not recommended to be used for long periods, namely, of more than one month, after its first opening, even if they comprise such anti-contamination closure systems.

Therefore, there is still the need to provide further preservative-free multiple-dose systems for ophthalmic formulations of anti-glaucoma agents, in particular of carbonic anhydrase inhibitors, whose sterility and microbiological integrity remains for long periods after the first opening of the dosage form, and wherein such sterility and microbiological integrity can be quantitatively assessed using tests which mimic the real-life usage conditions.

### Object of the invention

The object of the present invention is a multi-dose dispensing device filled with a preservative-free ophthalmic composition comprising a carbonic anhydrase inhibitor.

### Detailed description of the invention

The object of the present invention is a multi-dose dispensing device filled with a preservative-free ophthalmic composition comprising a carbonic anhydrase inhibitor, wherein:
- the ophthalmic composition is an aqueous composition having a viscosity comprised between 50 and 200 mPa·s;
- the multi-dose dispensing device comprises a container where the ophthalmic composition is housed and a nozzle, said nozzle comprising means for preventing microbial contamination;
characterized in that the composition remains microbiologically acceptable during use for a period of at least 90 days after the first opening of the container as assessed in a microbiological test comprising repeated microbial challenge with four microorganisms, wherein said four microorganisms consist of one gram-negative bacterium, one gram-positive bacterium, one fungus of the *Aspergillus* genus and *Candida albicans.*

The authors of the present invention have developed a preservative-free ophthalmic composition for glaucoma therapy comprising a carbonic anhydrase inhibitor in a multi-dose dispensing device, which remains microbiologically acceptable for at least 90 days after the first opening as assessed in a very demanding microbiological test which simulates the real-life usage conditions of the product by patients, including any possible misuse or inappropriate storage of the product.

Along the present description, as well as in the claims, the singular expressions, generally preceded by the articles "a", "an" or "the", are meant to include also the plural forms, unless the context clearly indicates otherwise. Furthermore, numeric values preceded by the term "about" are meant to include the exact stated value and also a certain variation around such value, namely a variation or ±5% of the stated amount. Numeric ranges defined by lower and upper endpoints are meant to include also said stated endpoints.

Along the present description, as well as in the claims, the concentration of a component in the ophthalmic composition expressed as % (w/v) means grams of said component per 100 ml of composition.

All terms as used herein in this application, unless otherwise stated, shall be understood in their ordinary meaning as known in the art. Other more specific definitions can be provided for certain terms as used herein, and are intended to apply uniformly through-out the specification and claims.

### Microbiological test

The microbiological test used to assess the microbiological acceptability of the composition comprises repeated microbial challenge with four microorganisms, wherein said four microorganisms consist of one gram-negative bacterium, one gram-positive bacterium, one fungus of the *Aspergillus* genus and *Candida albicans.*

In an embodiment, the gram-negative bacterium is *Pseudomonas aeruginosa.*

In an embodiment, the gram-positive bacterium is *Staphylococcus aureus.*

In an embodiment, the fungus of the *Aspergillus* genus is *Aspergillus brasiliensis.*

In an embodiment, the four microorganisms used in the microbiological test are *Pseudomonas aeruginosa, Staphylococcus aureus, Aspergillus brasiliensis* and *Candida albicans.*

The bacterial strains *Staphylococcus aureus* ATCC 6538 and *Pseudomonas aeruginosa* ATCC 9027, for example, can be used.

The fugal strains *Candida albicans* ATCC 10231 and *Aspergillus brasiliensis* ATCC 16404, for example, can be used.

The microbial challenge means that the tip of the nozzle is dipped in a contaminated challenge suspension comprising the stated microorganisms, preferably, using separated suspensions each containing one of said microorganisms. Thus, typically, the cap of the nozzle of each challenged sample is removed, the tip of the nozzle is immersed into the tested contaminated solution during about 5 seconds, then the system is removed from the contaminated suspension, and the cap is again placed on the nozzle. The sample can be stored at room temperature, without wiping the tip, until the next actuation.

Typically, several samples, i.e., several multi-dose dispensing devices filled with the preservative-free ophthalmic composition, are required to perform the microbial test, so that each sample is contaminated with a solution comprising only one of the four microorganisms. Therefore, several samples are used in parallel for separate challenges with every microorganism.

Said contaminated suspensions used for the microbiological challenge preferably contain at least 10⁶ colony forming units (CFUs) of each germ, typically about 10⁶ CFUs.

For mimicking the real-life conditions of usage of the ophthalmic composition and thus reproducing the possible risk of contamination due to misuse, the challenge with the different microorganisms is repeated several times along the test period, which is of at least 90 days, for example, between 90 and 100 days, preferably between 90 and 95 days.

Therefore, during said test period, the challenge with the microbial suspensions is repeated preferably at least 4 times, for example, 4, 5, 6, 7 or 8 times, preferably at least 5 times, and more preferably 6 times. Said challenges with the microorganisms preferably are evenly distributed along the testing period. For example, the first challenge is performed the first day of the test, the last challenge is performed between 10-20 days before the end of the test, for example about 14 days before the end of the test and the rest of challenges are allocated in between. Generally, the time period between each challenge ranges from 13 to 20 days.

Also, in order to mimic the real-life conditions of use, during the microbiological test period, one drop of the composition is normally dispensed every day. To that end, typically, the cap is removed from the nozzle, the dispenser is normally actuated once, tip downward, to deliver one drop of the composition and the tap is again replaced. As stated above, the samples are stored at room temperature, without wiping the tip, until next actuation. For the purposes of the test, for dispensing one drop "every day", it suffices to dispense one-drop, once daily, at least 5 days a week. For example, dispensing one-drop, once daily, 5 days a week, may be more convenient as no drop dispensing at the weekend is required. Alternatively, for the purposes of the test, for dispensing one drop "every day", it is also admissible to compensate not dispensing drops during weekend by dispensing two-drops, instead of one-drop, 2 days of the week, for dispensing a total of 7 drops per week.

The preservative-free ophthalmic composition of the present invention remains microbiologically acceptable during the test period. "Microbiologically acceptable", as used herein, means that the drops delivered and the composition inside the bottle fulfil certain acceptance criteria during all the test period, namely, low microbial bioburden of the delivered drops and no contamination (sterility) of the bottle content.

For assessing the sterility of the content, a sterility assay for each strain tested is performed at different time points during the test, according to European Pharmacopoeia 2.6.1. Preferably, four samples are used for each testing, at each time point and the sterility of the composition inside the container is checked. Typically, the composition inside the container is sampled using a syringe and is put onto a filter of 0.45 µm, then the filter is transferred to a culture medium, which typically is TSB or TRB, and the inoculum is incubated for about 14 days at 30-35°C (for TSB) or 20-25°C (for TRB). Then it is observed whether any microbial growth is detected. Sterility means absence of any microbial growth. The sterility is assessed at least 2 times during the test period, preferably at least 3 times, more preferably at least 4 times, and more preferably 4 times. Conveniently, the last sterility assay is performed about the last day of the test, while the other sterility checks may be performed at any time during the test period, distributed along said period, for example, the sterility assay may be performed 2-5 days after a challenge with the microorganisms, as disclosed above.

Bioburden testing for each microorganism is repeated several times during the testing period. Bioburden test is repeated at least 2 times, preferably at least 3 times and more preferably at least 4 times during the testing period. For fungi, each repetition of the bioburden testing is typically performed at one point after a challenge with the microorganism (i.e., contamination of the tip of the device, as disclosed above), typically about 14 days after the challenge. For bacteria, each repetition of the bioburden testing is typically performed at three time points after a challenge with the microorganism, typically, 24h, 3 days and 14 days after the microbial challenge.

An initial bioburden control test is also performed at the beginning of the test period (T0) for every microorganism. At least one sample per microorganism is tested, typically about 2-5 samples per microorganism. The samples are contaminated as described above, i.e., the tip of the nozzle is dipped in a contaminated challenge suspension comprising the microorganism. Immediately after contamination, the bioburden level is calculated ("initial bioburden level").

The bioburden test is performed according to well-known methods, for example as disclosed in Eur. Ph. Typically, 2 drops of the composition tested are delivered in a culture medium, the plates are incubated for 5 days at temperature 30-35°C, and then the number of colonies is counted to determine the colony forming units (CFUs).

The "low microbial burden" requirement is defined as follows:
- Log reduction of at least 1 for bacterial count about 24h after a challenge;
- No increase in number of viable microorganisms compared to initial bioburden level for bacterial count about 3 days after challenge;
- No increase in number of viable microorganisms compared to initial bioburden level for bacterial count about 14 days after challenge; and
- Log reduction of a least 1 for fungal count about 14 days after challenge.

### Multi-dose dispensing device

The "multi-dose dispensing device", as used herein, is understood as the whole primary package containing the preservative-free ophthalmic composition and typically comprises a container where the composition is housed, a nozzle which allows for the dropwise delivery of the composition, and a cap covering the nozzle.

The container of said dispensing device may be also indistinctly referred to herein as bottle, reservoir, or vessel. Said container is made of a flexible pharmaceutically acceptable packaging material, as are well-known in the art, for example, a polymer selected from the group consisting of polyethylene (PE), polypropylene (PP), polyethylene terephthalate (PET), polyvinyl chloride, acrylic resins, polystyrene, polymethylmethacrylate, nylon 6 and mixtures thereof. Preferably, the container is made of polypropylene (PP) or polyethylene (PE), preferably, low-density polyethylene (LDPE). These materials are widely commercially available from companies such as Dow or LyondellBasell. One preferred material for the container is low-density polyethylene (LDPE) Purell PE 1840H (LyondellBasell). The containers are manufactured according to well-known techniques, typically by injection blow moulding.

The volume of the container is suitably adapted to house the required volume of the ophthalmic composition. The amount of the composition is calculated for a treatment duration of at least 90 days, typically 90-100 days, i.e., which corresponds to about 3 months. Typically, the volume of the composition for such duration ranges from about 7 ml to about 15 ml, depending on the particular active ingredient and the particular formulation.

The nozzle of the multi-dose dispensing device allows for the dropwise delivery of the formulation and comprises means for preventing microbial contamination of said composition. The prevention of the microbial contamination relates both during the shelf-life, i.e., during storage before being used, and, particularly, also during use after the first opening of the dispensing device.

The prevention of microbial contamination during shelf-life is conventionally performed by shielding the contents of the container from the environment, as is standard practice in the art, typically, by airtight assembling of the container, the nozzle and the cap; the cap typically tightly engages the nozzle and protects the nozzle tip. Furthermore, the cap may optionally include a collar that is broken and removed after the first opening the dispenser and acts as a guarantee tamper-evident seal, to ensure that the device has not been opened before the first use.

On the other hand, the nozzle comprises means for preventing the microbial contamination also after the first opening of the dispensing device, while the composition is in use. During this period, the risk of contamination increases due to possible misuse, including contacting the tip of the nozzle with contaminated surfaces, for example, by direct contact with the eye surface, or due to incorrect closure of the device after use. Such means for preventing the microbial contamination during use consist in a closure system fitted in the nozzle of the device, which acts as a barrier, avoiding the entry of pathogens inside the device.

Several closure systems for preservative-free multidose devices (PFMD) are known in the art and are suitable for the present invention. They are typically based either on a pump system or on a non-return valve combined with a filter for the compensating air entering into the container, for example as disclosed in Yoon et al., Packaging Development: Multi-dose container closure for preservative free products, extractable/leachables from packaging, new technologies, in: Neervannan, S., Kompella, U.B. (eds) Ophthalmic Product Development. AAPS Advances in the Pharmaceutical Sciences Series, vol 37. Springer, Ophthalmic Product Development, 229-245.

Suitable examples of commercially available closure systems for PFMD delivery devices based on a pump are COMOD^{®} (Ursapharm) and 3K^{®} (Adelphi Healthcare Packaging). In said pump systems, typically, when the pump is activated, the product contained in the dosing chamber of the pump is subjected to pressure, which opens the outlet valve and releases a drop. Upon completion of the dosing process, the valve closes immediately afterwards and therefore backflow of the product is prevented. The pump is reset to its initial position and a further aliquot of the product is drawn from the container into the dosing chamber. In the 3K^{®} system, there is airtight sachet inside the container, where the composition is packaged, which at no point comes into contact with the surrounding air. When the pump system is activated, a stream of air flows into the space between the inner sachet and the container wall to equalise the pressure, the negative pressure created pulls the flexible inner sachet, stays sealed and the liquid is protected against contamination from the air. In the COMOD^{®} system, the vacuum within the container is compensated by the subsequent inflow of air, which passes through a special filter matrix.

On the other hand, suitable examples of commercially available closure systems for PFMD delivery devices based on non-return valves combined with filters for the incoming air are Novelia^{®} (Nemera) and OSD^{®} (Aptar).

In OSD^{®} and Novelia^{®} systems, there is a non-return (one-way) valve located in the nozzle tip, which provides a continuous barrier to the external environment. Therefore, when the container is squeezed, the tip opens and the product is delivered, and then the tip closes again, so that the sealing mechanism protecting the orifice avoids that contaminated liquid can re-enter the container. In OSD^{®}, filtered ambient air enters the container allowing pressure re-equilibration, and passes a 0.2 µm filter membrane. In Novelia^{®}, the intake of air to re-equilibrate the pressure into the bottle takes place via a venting system comprising a silicone membrane, in the form of a silicone plug, to filter the returning air, which is separated from the non-return valve. The intermolecular distance in this silicone membrane is of the order of nanometers, allowing the passage of air, but preventing the passage of any liquid or solid, including bacteria.

In any of the above-described closure systems, the anti-microbial effectiveness may be enhanced by the use of a silver releasing agent embedded into the top of the nozzle that is able to release silver ions, a well-known antibacterial agent. For example, Bactiglas^{®} is a commercially available material, which is able to release silver ions in a controlled and sustained way. This silver releasing agent may be embedded in the tip of the nozzle or on the part of the cap in contact with said tip or, preferably, in both. In this way, the possible residual drop of the product which remains in the tip of the dispenser stays in contact with such silver anti-microbial agent.

The nozzle is made of different materials. The structural components are typically made of high-density polypropylene (HDPE), while other parts may be made of different materials. For example, in Novelia^{®} system, the valve and the plug for filtering the air are made of silicone.

The cap covers the nozzle of the device, and is typically a screw cap made of HDPE. The internal part of the top of the cap, which is in contact with the orifice of the nozzle, as disclosed above, can contain a silver releasing agent.

### Carbonic anhydrase inhibitor

The preservative-free ophthalmic composition contained in the multi-dose dispensing device according to the present invention, comprises a carbonic anhydrase inhibitor (CAI) as active anti-glaucoma agent, optionally in combination with a second, or further, anti-glaucoma agents.

Carbonic anhydrase inhibitors (CAIs), as are well-known in the art, are substances that selectively inhibit carbonic anhydrase isoenzyme II in the ciliary epithelium, leading to decreased aqueous production, thereby lowering intraocular pressure.

The CAI is preferably selected from dorzolamide, brinzolamide and pharmaceutically acceptable salts thereof. Both substances are well-known in the art and are widely available from commercial sources.

In one preferred embodiment, the CAI inhibitor is dorzolamide or a pharmaceutically acceptable salt thereof. In one particularly preferred embodiment, the CAI inhibitor is dorzolamide hydrochloride.

### Ophthalmic compositions

The multi-dose dispensing device according to the present invention comprises a "preservative-free" ophthalmic composition. The term "preservative-free" means that the ophthalmic composition of the present invention does not comprise any preservative conventionally used for the preservation of ophthalmic compositions, for example, the composition does not contain quaternary ammonium compounds, such as benzalkonium chloride (BAK). Other pharmaceutically acceptable preservatives for ophthalmic solutions, which are also excluded from the present composition, are, for example, parabens (such as ethylparaben or butylparaben), chlorhexidine, benzethonium chloride, or sorbic acid or salts (such as potassium sorbate), among others.

The skilled person will appreciate that any substance that is known to provide a preservative effect should be excluded in this context, irrespective of any other function that the substance could simultaneously have in the liquid preparation.

The ophthalmic composition of the invention comprises the carbonic anhydrase inhibitor, as disclosed above.

Preferably, the amount of the carbonic anhydrase inhibitor in the ophthalmic composition ranges from 0.5% to 4.0% (w/v), more preferably from 0.6% to 3.0% (w/v), and still more preferably from 0.75% to 2.25% (w/v). When the carbonic anhydrase inhibitor substance is in the form of a pharmaceutically acceptable salt thereof, said concentration is generally expressed as the equivalent concentration of the non-salified substance.

In an embodiment, the carbonic anhydrase inhibitor is dorzolamide or a pharmaceutically acceptable salt thereof, preferably dorzolamide hydrochloride, in a concentration ranging from 0.75% to 4.0% (w/v), preferably from 1.0% to 3.0% (w/v), more preferably from 1.5% to 2.5% (w/v), still more preferably from 1.8% to 2.2% (w/v), and still more preferably in a concentration of about 2.0% (w/v). Said concentration, when dorzolamide is in the form of a pharmaceutically acceptable salt thereof, is expressed as equivalent dorzolamide concentration.

Optionally, the ophthalmic composition may contain a second anti-glaucoma active ingredient, as a fixed-dose combination.

In an embodiment, the ophthalmic composition contains a beta-blocker as a second anti-glaucoma active ingredient. Beta-blockers suitable to be used in combination with the carbonic anhydrase inhibitor are, for example, timolol, levobunolol, metipranolol, carteolol or betaxolol, or a pharmaceutically acceptable salt thereof. One preferred beta-blocker is timolol, or an acceptable salt thereof. One preferred salt of timolol is timolol maleate.

When the ophthalmic composition comprises timolol, or a pharmaceutically acceptable salt thereof, as additional active ingredient, it is generally in an amount ranging from 0.1% to 1.0% (w/v), preferably from 0.2% to 0.8% (w/v), more preferably from 0.3% to 0.7% (w/v), still more preferably from 0.4% to 0.6% (w/v), and still more preferably about 0.5% (w/v). Said concentration, when timolol is in the form of a pharmaceutically acceptable salt thereof, is expressed as equivalent timolol concentration.

In another embodiment, the ophthalmic composition contains an alpha-2 adrenergic receptor agonist as a second anti-glaucoma active ingredient.

Examples of alpha-2 adrenergic receptor agonists suitable to be used in combination with the carbonic anhydrase inhibitor are, for example, apraclonidine, brimonidine, and pharmaceutically acceptable salts thereof. One preferred alpha-2 adrenergic receptor agonists is brimonidine, or an acceptable salt thereof. One preferred salt of brimonidine is brimonidine tartrate.

When the ophthalmic composition comprises brimonidine, or a pharmaceutically acceptable salt thereof, as additional anti-glaucoma active ingredient, it is generally in an amount ranging from 0.05% to 0.5% (w/v), preferably from 0.08% to 0.4% (w/v), more preferably from 0.1% to 0.3% (w/v), and still more preferably in an amount of about 0.2% (w/v). Said concentration, irrespective of the brimonidine form used, either as free base or a pharmaceutically acceptable salt thereof, is generally expressed as equivalent brimonidine tartrate concentration.

The ophthalmic composition is an aqueous composition, typically selected from a solution and a suspension, and preferably is a solution.

Said aqueous ophthalmic composition is characterized in that it has a viscosity comprised between 50 and 200 mPa·s.

In an embodiment, the viscosity is comprised between 60 and 180 mPa·s, preferably comprised between 70 and 175 mPa·s.

The viscosity of the ophthalmic composition may be measured according to standard procedures, typically, using a viscosimeter, as are well-known in the art.

The ophthalmic composition of the present invention is aqueous, i.e., the major solvent or vehicle is water. Typically, water for injection (WFI) is used as solvent or vehicle, according to the quality standards stated in the Pharmacopoeias. WFI is generally obtained by water purification using procedures as distillation, deionization, reverse-osmosis, membrane filtration or combination thereof.

The aqueous ophthalmic composition according to the invention may also comprise conventional pharmaceutically acceptable excipients used in ophthalmic compositions. Said excipients are well-known in the art and are disclosed, for example, in the book "Handbook of Pharmaceutical Excipients" (8th edition, 2017, Rowe et a/., editors, Pharmaceutical Press).

Common excipients that can be included in the ophthalmic composition of the invention are, for example, viscosizing agents, tonicity agents, buffering agents, cosolvents, surfactants, antioxidants, or mixtures thereof.

The aqueous ophthalmic composition typically comprises a viscosizing agent in order to adjust the viscosity of the composition. Among the viscosizing agents suitable for being used in the composition of the invention are, for example, sodium carboxymethylcellulose, hydroxyethyl cellulose, hydroxypropyl cellulose, hydroxymethyl cellulose, hydroxypropyl methylcellulose, methylcellulose, carbomers, polyvinylpyrrolidone, polyvinyl alcohol, polyoxyethylene, polycarbophil, hyaluronic acid or its sodium salt, or mixtures thereof. Preferably, the viscosizing agent is selected from sodium carboxymethylcellulose, hydroxyethyl cellulose, polyvinylpyrrolidone, and mixtures thereof.

When the ophthalmic composition comprises a viscosizing agent, the concentration used is adjusted so as to provide a viscosity value in the claimed range, i.e., 50-200 mPa·s.

The ophthalmic composition of the invention may optionally comprise a tonicity agent. Tonicity agents, as is well-known in the art, are substances added to produce aqueous compositions which are approximately isotonic relative normal tears, in order to reduce pain and tissue irritation on application of the composition. Suitable osmolality values for the ophthalmic composition are comprised between about 250 and about 320 mOsm/kg. Suitable tonicity agents added to adjust the osmolality of the solution are, for example, sodium chloride, potassium chloride, calcium chloride, propylene glycol, glycerol, sorbitol or mannitol, or mixtures thereof. Preferably, the tonicity agent is selected from sodium chloride, mannitol and mixtures thereof.

The osmolality can be measured using known methods, typically using an osmometer.

The ophthalmic composition of the invention may also contain a buffering agent. As is well-known in the art, a buffering agent is a substance or substance combination, generally consisting of an acid and its conjugate base, capable in solution of neutralizing both acids and bases and thereby maintaining stable the pH of the solution. The preparation of buffering agents is well known. Thus, for example, the phosphate buffer is typically prepared as a mixture of the dihydrogen phosphate ion and the hydrogen phosphate ion, for example using the sodium dihydrogen phosphate salt (NaH₂PO₄, or monosodium phosphate) and the sodium hydrogen phosphate salt (Na₂HPO₄, or disodium phosphate). Analogously, the acetate buffer can be prepared with acetic acid and sodium acetate; the citrate buffer can be prepared with citric acid and sodium citrate; or the borate buffer can be prepared with boric acid and disodium tetraborate, for example. Mixed buffers are also suitable, for example the citrate-phosphate buffer, prepared with citric acid and disodium hydrogen phosphate.

For example, the composition of the invention comprises a buffering agent selected from phosphate buffer, borate buffer, citrate buffer and citrate-phosphate buffer. In an embodiment, the composition comprises citrate buffer.

The pH of the ophthalmic solution is adjusted to a value ranging from about 4.5 to about 7.5, preferably ranging from 5.0 to 6.5. To further adjust the pH value, usually an acid and/or base are added to the composition, typically, hydrochloric acid and/or sodium hydroxide.

The pH of the composition is measured using known procedures, typically, using a pH-meter.

The aqueous ophthalmic composition may optionally comprise an organic cosolvent. Suitable cosolvents are, for example, propylene glycol or polyethylene glycol, among others. If the composition comprises a cosolvent, it is generally in an amount in the range 0.1-1.0% (w/v).

The composition may optionally contain a surfactant. Surfactants, as is well-known in the art, are compounds that lower the surface tension between two liquids or between a liquid and a solid, and they may be non-ionic, anionic or cationic.

When the composition of the invention comprises a surfactant, it is preferably a non-ionic surfactant, for example, a polyoxyethylene fatty alcohol ether; or a polyoxyethylene fatty acid ester, such as polyoxyethylene sorbitan, mono- or polyester or polyoxyethylene glycerol, mono- or polyester; or a fatty acid ester, such as a sorbitan fatty acid ester, or a glycerol fatty acid ester; or poloxamers, among others, or mixtures thereof. For example, the non-ionic surfactant may be selected from the group consisting of polysorbate 80, polysorbate 60, polysorbate 40, polysorbate 20, polysorbate 65, polyoxyethylene sorbitan trioleate, polyoxyethylene (10) hydrogenated castor oil, polyoxyethylene (40) hydrogenated castor oil, polyoxyethylene (50) hydrogenated castor oil, polyoxyethylene (60) hydrogenated castor oil, polyoxyethylene (40) stearate, and mixtures thereof.

When the ophthalmic composition comprises a surfactant, it is generally in an amount comprised between 0.01% (w/v) and 1.0% (w/), preferably comprised between 0.01% (w/v) and 0.75% (w/v), and more preferably comprised between 0.04% (w/v) and 0.60% (w/v).

The composition may optionally contain an antioxidant. Antioxidants, as is well-known in the art, are substances that prevent the oxidation of other molecules. For example, ethylenediamine tetraacetic acid (also known as edetic acid) and salts thereof (for example, disodium edetate) is a chelating agent which also has antioxidant properties, as it prevents autoxidation reactions. If the composition comprises an antioxidant, it is generally in an amount comprised in the range 0.005-0.1% (w/v), preferably in the range 0.01-0.05% (w/v).

The ophthalmic composition of the invention is prepared according to conventional methods, typically, by dissolving or suspending the carbonic anhydrase inhibitor, optionally an additional active ingredient, and any optional excipients in water, adjusting the pH value if needed, and sterilizing, either by aseptic filtration through a 0.22-µm filter or by autoclaving.

A suitable volume of the composition is calculated which is sufficient for a 3-month therapy, considering the concentration of the carbonic anhydrase inhibitor in the composition and the recommended therapeutic dose, as is well-known for the skilled in the art. For example, for dosage regimes requiring one drop, two drops or three drops daily, approximate volumes of about 5, 10 and 14 ml, respectively, of the composition are typically required for a 3-month therapy.

A suitable container is selected for the required volume of the composition, and the composition is filled therein. The corresponding nozzle is assembled, typically screwed, with a cap covering the nozzle.

As shown in the examples, the preservative-free carbonic anhydrase inhibitor ophthalmic composition in the multi-dose dispensing device, according to the present invention, remains microbiologically acceptable for a period of at least 3 months and is therefore particularly suitable to be used in therapy, in particular, for the treatment of elevated intraocular pressure in patients with glaucoma, for example, with open-angle glaucoma.

In an embodiment, the preservative-free ophthalmic composition contained within the dispensing device of the invention is an aqueous solution comprising:
- dorzolamide, or a pharmaceutically acceptable salt thereof, as active ingredient;
- optionally, a second anti-glaucoma agent, preferably selected from timolol, or a pharmaceutically acceptable salt thereof, and brimonidine, or a pharmaceutically acceptable salt thereof;
- a viscosizing agent, preferably selected from sodium carboxymethylcellulose, hydroxyethyl cellulose, hydroxypropyl cellulose, hydroxylmethyl cellulose, hydroxypropyl methylcellulose, methylcellulose, carbomers, polyvinylpyrrolidone, polyvinyl alcohol, polyoxyethylene, polycarbophil, hyaluronic acid or its sodium salt, and mixtures thereof, more preferably selected from sodium carboxymethylcellulose, hydroxyethyl cellulose, polyvinylpyrrolidone, and mixtures thereof;
- a tonicity agent, preferably selected from sodium chloride, potassium chloride, calcium chloride, propylene glycol, glycerol, sorbitol, mannitol and mixtures thereof, more preferably selected from sodium chloride, mannitol and mixtures thereof, and still more preferably the tonicity agent is mannitol;
- a buffering agent, preferably citrate buffer;
- optionally, a pH adjusting agent such as hydrochloric acid and/or sodium hydroxide to adjust the pH of the solution to a value in the range 4.5-7.5, preferably in the range 5,0-6,5; and
- water, as solvent;
wherein said ingredients are used according to the specifications and preferred concentrations as disclosed above. In a particular embodiment, the composition essentially consists of the above listed ingredients.

In an embodiment, the preservative-free ophthalmic composition contained within the dispensing device of the invention is an aqueous solution comprising dorzolamide, or a pharmaceutically acceptable salt thereof, as active ingredient, a viscosizing agent, preferably hydroxyethyl cellulose, a tonicity agent, and a buffering agent.

In a preferred embodiment, said preservative-free ophthalmic composition is an aqueous solution comprising:
- dorzolamide, or a pharmaceutically acceptable salt thereof, preferably dorzolamide hydrochloride, in a concentration comprised in the range 0.75-4.0% (w/v), preferably in the range 1.0-3.0% (w/v), more preferably in the range 1.5-2.5% (w/v), still more preferably in the range 1.8-2.2% (w/v), still more preferably in a concentration of about 2.0% (w/v), and still more preferably in a concentration of 2.0% (w/v), wherein the concentration is expressed as the concentration of dorzolamide;
- hydroxyethyl cellulose in a concentration comprised in the range 0.1-1.5% (w/v), preferably in the range 0.2-1.0% (w/v), more preferably in the range 0.3-0.7% (w/v), and still more preferably in the range 0.4-0.5% (w/v);
- a tonicity agent in an amount sufficient to adjust the osmolality to a value comprised in the range 250-320 mOsmol/kg, preferably wherein the tonicity agent is selected from the group consisting of sodium chloride, potassium chloride, calcium chloride, propylene glycol, glycerol, sorbitol, mannitol and mixtures thereof, more preferably selected from sodium chloride, mannitol and mixtures thereof, and more preferably the tonicity agent is mannitol;
- an ophthalmically-acceptable buffering agent, preferably, citrate buffer, more preferably sodium citrate, still more preferably sodium citrate in a concentration comprised in the range 0.05-1.00% (w/v); more preferably in the range 0.10-0.80% (w/v), still more preferably in the range 0.20-0.60% (w/v), and still more preferably in the range 0.25-0.40% (w/v);
- optionally, a pH adjusting agent such as hydrochloric acid and/or sodium hydroxide to adjust the pH of the solution to a value in the range 4.5-7.5, preferably in the range 5.5-6.5; and
- water, as solvent;
wherein the viscosity of the composition is preferably comprised between 75 and 160 mPa·s.

In a more specific embodiment, the ophthalmic composition contained within the dispensing device of the invention is an aqueous solution, which essentially consists of dorzolamide, or a pharmaceutically acceptable salt thereof, hydroxyethyl cellulose, a tonicity agent, a buffering agent, optionally a pH adjusting agent, and water as solvent, according to the preferences disclosed above.

In another embodiment, the preservative-free ophthalmic composition contained within the dispensing device of the invention is an aqueous solution comprising dorzolamide, or a pharmaceutically acceptable salt thereof, and timolol, or a pharmaceutically acceptable salt thereof, as active ingredients, a viscosizing agent, preferably hydroxyethyl cellulose, a tonicity agent, and a buffering agent.

In a preferred embodiment, said preservative-free ophthalmic composition is an aqueous solution comprising:
- dorzolamide, or a pharmaceutically acceptable salt thereof, preferably dorzolamide hydrochloride, in a concentration comprised in the range 0.75-4.0% (w/v), preferably in the range 1.0-3.0% (w/v), more preferably in the range 1.5-2.5% (w/v), still more preferably in the range 1.8-2.2% (w/v), still more preferably in a concentration of about 2.0% (w/v), and still more preferably in a concentration of 2.0% (w/v), wherein the concentration is expressed as the concentration of dorzolamide;
- timolol, or a pharmaceutically acceptable salt thereof, preferably timolol maleate, in a concentration comprised in the range 0.1-1.0% (w/v), preferably in the range 0.2-0.8% (w/v), more preferably in the range 0.3-0.7% (w/v), still more preferably in the range 0.4-0.6% (w/v), still more preferably in a concentration of about 0.5% (w/v), and still more preferably in a concentration of 0.5% (w/v), expressed as the concentration of timolol;
- hydroxyethyl cellulose in a concentration comprised in the range 0.1-1.5% (w/v), preferably in the range 0.2-1.0% (w/v), more preferably in the range 0.3-0.7% (w/v), and still more preferably in the range 0.4-0.5% (w/v);
- a tonicity agent in an amount sufficient to adjust the osmolality to a value comprised in the range 250-320 mOsmol/kg, preferably wherein the tonicity agent is selected from the group consisting of sodium chloride, potassium chloride, calcium chloride, propylene glycol, glycerol, sorbitol, mannitol and mixtures thereof, more preferably selected from sodium chloride, mannitol and mixtures thereof, and more preferably the tonicity agent is mannitol;
- an ophthalmically-acceptable buffering agent, preferably, citrate buffer, more preferably sodium citrate, still more preferably sodium citrate in a concentration comprised in the range 0.05-1.00% (w/v); more preferably in the range 0.10-0.80% (w/v), still more preferably in the range 0.20-0.60% (w/v), and still more preferably in the range 0.25-0.40% (w/v);
- optionally, a pH adjusting agent such as hydrochloric acid and/or sodium hydroxide to adjust the pH of the solution to a value in the range 4.5-7.5, preferably in the range 5.5-6.5; and
- water, as solvent;
wherein the viscosity of the composition is preferably comprised between 75 and 160 mPa·s.

In a more specific embodiment, the ophthalmic composition contained within the dispensing device of the invention is an aqueous solution which essentially consists of dorzolamide, or a pharmaceutically acceptable salt thereof, timolol, or a pharmaceutically acceptable salt thereof, hydroxyethyl cellulose, a tonicity agent, a buffering agent, optionally a pH adjusting agent, and water as solvent, according to the preferences disclosed above.

In another embodiment, the preservative-free ophthalmic composition contained within the dispensing device of the invention is an aqueous solution comprising dorzolamide, or a pharmaceutically acceptable salt thereof, and brimonidine, or a pharmaceutically acceptable salt thereof, as active ingredients, a viscosizing agent, preferably selected from sodium carboxymethylcellulose, hydroxyethyl cellulose, polyvinylpyrrolidone, and mixtures thereof, a tonicity agent, and a buffering agent.

In a preferred embodiment, said preservative-free ophthalmic composition is an aqueous solution comprising:
- dorzolamide, or a pharmaceutically acceptable salt thereof, preferably dorzolamide hydrochloride, in a concentration comprised in the range 0.75-4.0% (w/v), preferably in the range 1.0-3.0% (w/v), more preferably in the range 1.5-2.5% (w/v), still more preferably in the range 1.8-2.2% (w/v), still more preferably in a concentration of about 2.0% (w/v), and still more preferably in a concentration of 2.0% (w/v), wherein the concentration is expressed as the concentration of dorzolamide;
- brimonidine, or a pharmaceutically acceptable salt thereof, preferably brimonidine tartrate, in a concentration comprised in the range 0.05-0.5% (w/v), preferably in the range 0.08-0.4% (w/v), more preferably in the range 0.1-0.3% (w/v), still more preferably in a concentration of about 0.2% (w/v), and still more preferably in a concentration of 0.2% (w/v), expressed as the equivalent concentration of brimonidine tartrate;
- a viscosizing agent which is either:
   a) sodium carboxymethylcellulose in a concentration comprised in the range 0.1-2.5% (w/v), preferably in the range 0.5-2.0% (w/v), and more preferably in the range 1.0-1.5% (w/v); or
   b) hydroxyethyl cellulose in a concentration comprised in the range 0.1-1.5% (w/v), preferably in the range 0.2-1.0% (w/v), more preferably in the range 0.3-0.7% (w/v), and more preferably in the range 0.4-0.5% (w/v); or
   c) polyvinylpyrrolidone in a concentration comprised in the range 1.0-8.0% (w/v), preferably in the range 3.0-7.0% (w/v), and more preferably in the range 5.0-6.5% (w/v);
- a tonicity agent in an amount sufficient to adjust the osmolality to a value comprised in the range 250-320 mOsmol/kg, preferably wherein the tonicity agent is selected from the group consisting of sodium chloride, potassium chloride, calcium chloride, propylene glycol, glycerol, sorbitol, mannitol and mixtures thereof, more preferably selected from sodium chloride, mannitol and mixtures thereof, and more preferably the tonicity agent is mannitol;
- an ophthalmically-acceptable buffering agent, preferably, citrate buffer, more preferably sodium citrate, still more preferably sodium citrate in a concentration comprised in the range 0.05-1.00% (w/v); more preferably in the range 0.10-0.80% (w/v), still more preferably in the range 0.20-0.60% (w/v), and still more preferably in the range 0.25-0.40% (w/v);
- optionally, a pH adjusting agent such as hydrochloric acid and/or sodium hydroxide to adjust the pH of the solution to a value in the range 4.5-7.5, preferably in the range 5.5-6.5; and
- water, as solvent;
wherein the viscosity of the composition is preferably comprised between 75 and 160 mPa·s.

In a more specific embodiment, the ophthalmic composition contained within the dispensing device of the invention is an aqueous solution which essentially consists of dorzolamide, or a pharmaceutically acceptable salt thereof, brimonidine, or a pharmaceutically acceptable salt thereof, a viscosizing agent selected from sodium carboxymethylcellulose, hydroxyethyl cellulose and polyvinylpyrrolidone, a tonicity agent, a buffering agent, optionally a pH adjusting agent, and water as solvent, according to the preferences disclosed above.

The present invention relates to the following embodiments:
1. A multi-dose dispensing device filled with a preservative-free ophthalmic composition comprising a carbonic anhydrase inhibitor, wherein:
   - the ophthalmic composition is an aqueous composition having a viscosity comprised between 50 and 200 mPa·s;
   - the multi-dose dispensing device comprises a container where the ophthalmic composition is housed and a nozzle, said nozzle comprising means for preventing microbial contamination;
   characterized in that the composition remains microbiologically acceptable during use for a period of at least 90 days after the first opening of the container as assessed in a microbiological test comprising repeated microbial challenge with four microorganisms, wherein said four microorganisms consist of one gram-negative bacterium, one gram-positive bacterium, one fungus of the *Aspergillus* genus and *Candida albicans.*
2. The multi-dose dispensing device according to embodiment 1, characterized in that the gram-negative bacterium is *Pseudomonas aeruginosa.*
3. The multi-dose dispensing device according to embodiment 1 or 2, characterized in that the gram-positive bacterium is *Staphylococcus aureus.*
4. The multi-dose dispensing device according to any one of embodiments 1 to 3, characterized in that the fungus of the *Aspergillus* genus is *Aspergillus brasiliensis.*
5. The multi-dose dispensing device according to any one of embodiments 1 to 4, characterized in that the microbiological challenge is performed with suspensions of each microorganism containing about 10⁶ colony forming units (CFUs) of each germ.
6. The multi-dose dispensing device according to any one of embodiments 1 to 5, characterized in that the duration of the microbiological test is comprised between 90 and 100 days.
7. The multi-dose dispensing device according to any one of embodiments 1 to 6, characterized in that the microbial challenge is repeated at least 4 times during the testing period, preferably at least 5 times, more preferably at least 6 times, and more preferably 6 times.
8. The multi-dose dispensing device according to any one of embodiments 1 to 7, characterized in that during the testing period one drop of the composition is normally dispensed every day.
9. The multi-dose dispensing device according to any one of embodiments 1 to 8, characterized in that the means for preventing microbiological contamination consist in a closure system fitted in the nozzle.
10. The multi-dose dispensing device according to embodiment 9, characterized in that the closure system is selected from a pump system and a non-return valve combined with a filter for the air returning to the container.
11. The multi-dose dispensing device according to any one of embodiments 1 to 10, characterized in that it comprises a silver releasing antibacterial agent.
12. The multi-dose dispensing device according to any one of embodiments 1 to 11, characterized in that the carbonic anhydrase inhibitor is selected from dorzolamide, brinzolamide, and pharmaceutically acceptable salts thereof.
13. The multi-dose dispensing device according to any one of embodiments 1 to 12, characterized in that the amount the carbonic anhydrase inhibitor in the ophthalmic composition ranges from 0.5% to 4.0% (w/v), preferably from 0.6% to 3.0% (w/v), and more preferably from 0.75% to 2.25% (w/v).
14. The multi-dose dispensing device according to any one of embodiments 1 to 13, characterized in that the carbonic anhydrase inhibitor is dorzolamide or a pharmaceutically acceptable salt thereof, preferably is dorzolamide hydrochloride.
15. The multi-dose dispensing device according to embodiment 14, characterized in that the amount of dorzolamide or a pharmaceutically acceptable salt thereof in the ophthalmic composition ranges from 0.75% to 4.0% (w/v), preferably from 1.0% to 3.0% (w/v), more preferably from 1.5% to 2.5% (w/v), still more preferably from 1.8% to 2.2% (w/v), and still more preferably is about 2.0% (w/v), expressed as concentration of dorzolamide.
16. The multi-dose dispensing device according to any one of embodiments 1 to 15, characterized in that the ophthalmic composition also comprises a second anti-glaucoma active ingredient, which is preferably selected from a beta-blocker and an alpha-2 adrenergic receptor agonist.
17. The multi-dose dispensing device according to embodiment 16, characterized in that the ophthalmic composition comprises a beta-blocker selected from timolol, levobunolol, metipranolol, carteolol or betaxolol, or a pharmaceutically acceptable salt thereof.
18. The multi-dose dispensing device according to embodiment 17, characterized in that the beta-blocker is timolol or a pharmaceutically acceptable salt thereof, in an amount ranging from 0.1% to 1.0% (w/v), preferably from 0.2% to 0.8% (w/v), more preferably from 0.3% to 0.7% (w/v), still more preferably from 0.4% to 0.6% (w/v), and still more preferably in an amount of about 0.5% (w/v).
19. The multi-dose dispensing device according to embodiment 16, characterized in that the ophthalmic composition comprises an alpha-2 adrenergic receptor agonist selected from apraclonidine, brimonidine and pharmaceutically acceptable salts thereof.
20. The multi-dose dispensing device according to embodiment 19, characterized in that the alpha-2 adrenergic receptor agonist is brimonidine or a pharmaceutically acceptable salt thereof, preferably brimonidine tartrate, in an amount ranging from 0.05% to 0.5% (w/v), preferably from 0.08% to 0.4% (w/v), more preferably from 0.1% to 0.3% (w/v), and still more preferably in an amount of about 0.2% (w/v), expressed as equivalent brimonidine tartrate concentration.
21. The multi-dose dispensing device according to any one of embodiments 1 to 20, characterized in that the aqueous composition is selected from a solution and a suspension.
22. The multi-dose dispensing device according to embodiment 21, characterized in that the aqueous composition is a solution.
23. The multi-dose dispensing device according to any one of embodiments 1 to 22, characterized in that the viscosity of the composition is comprised between 60 and 180 mPa·s., preferably comprised between 70 and 175 mPa·s.
24. The muti-dose dispensing device according to any one of embodiments 1 to 23, characterized in that the ophthalmic composition additionally comprises a viscosizing agent selected from sodium carboxymethylcellulose, hydroxyethyl cellulose, hydroxypropyl cellulose, hydroxylmethyl cellulose, hydroxypropyl methylcellulose, methylcellulose, carbomers, polyvinylpyrrolidone, polyvinyl alcohol, polyoxyethylene, polycarbophil, hyaluronic acid or its sodium salt, and mixtures thereof, preferably selected from sodium carboxymethylcellulose, hydroxyethyl cellulose, polyvinylpyrrolidone, and mixtures thereof.
25. The multi-dose dispensing device according to any one of embodiments 1 to 24, characterized in that the ophthalmic composition additionally comprises a tonicity agent to adjust the osmolality of the composition to a value comprised between 250 and 320 mOsm/kg.
26. The multi-dose dispensing device according to embodiment 25, characterized in that the tonicity agent is selected from the group consisting of sodium chloride, potassium chloride, calcium chloride, propylene glycol, glycerol, sorbitol, mannitol and mixtures thereof, preferably is selected from sodium chloride, mannitol, and mixtures thereof, and more preferably is mannitol.
27. The multi-dose dispensing device according to any one of embodiments 1 to 26, characterized in that the composition comprises a buffering agent, preferably selected from phosphate buffer, borate buffer, citrate buffer and citrate-phosphate buffer and optionally hydrochloric acid and/or sodium hydroxide to adjust the pH of the composition to a value in the range 4.5-7.5.

### Examples

### Example 1 Preparation of bulk aqueous ophthalmic solution comprising dorzolamide

A solution was prepared with the ingredients listed in the following table:

| **Substance** | **% w/v (g in 100 ml)** |
|---|---|
| Dorzolamide (as dorzolamide HCI) | 2.000 (2.226) |
| Hydroxyethylcellulose (grade 1,500-2,500 mPa.s) | 0.475 |
| Mannitol | 2.30 |
| Sodium citrate | 0.294 |
| NaOH/HCI | q.s. pH=5.0-6.0 |
| Purified water | q.s. |

All the ingredients were dissolved in purified water, and the pH of the solution was finally adjusted with NaOH and/or HCI.

The viscosity of the composition was 70-160 mPa·s, measured at 20°C using a Brookfield viscosimeter.

### Example 2 Preparation a multidose ophthalmic composition comprising dorzolamide suitable for 3 months usage

The solution prepared in Example 1 was sterilized by filtration through a 0.22 µm filter. 14 ml of the sterile solution were introduced in a sterile white LDPE bottle (15 ml), having a Novelia^{®} nozzle with a tamper-proof HDPE screw cap, comprising a silver agent (Bactiglas^{®}) on the tip of the nozzle and on the inner part of the cap contacting such tip.

The multidose dispensing device comprising the dorzolamide composition was subjected to a microbiological test to assess microbiological compliance during use for a period of 91 days.

To that end, 98 units of the above product were used, i.e., 96 multidose delivery devices comprising 14 ml of the dorzolamide solution, as disclosed above.

The microbial challenge was performed using the following microorganisms:
- *Pseudomonas aeruginosa* ATCC 9027 at 10⁶ cfu/mL (B1-PA)
- *Staphylococcus aureus* ATCC 6538 at 10⁶ cfu/mL (B2-SA)
- *Candida albicans* ATCC 10231 at 10⁶ cfu/mL (F1-CA)
- *Aspergillus brasiliensis* ATCC 16404 at 10⁶ cfu/mL (F2-AB).

The samples were numbered and distributed as follows for the microbiological test:

| | Sample number: | |
|---|---|---|
| **Contaminated with:** | **For bioburden test** | **For sterility test** |
| B1-PA | 1,2,3,4, 81-84 (T0) | 17-20, 33-36, 49-52, 65-68, 81-84 (T0) |
| B2-SA | 5,6,7,8, 85-89 (T0) | 21-24, 37-40, 53-56, 69-72, 85-89 (T0) |
| F1-CA | 9,10,11,12, 90-94 (T0) | 25-28, 41-44, 57-60, 73-76, 90-94 (T0) |
| F2-AB | 13,14,15,16, 95-98 (T0) | 29-32, 45-48, 61-64, 77-80, 95-98 (T0) |

Samples 1-80 were used for bioburden and sterility testing along the 91-day testing period. Samples 81-98 were used for checking initial (T0) bioburden and sterility. For testing the initial bioburden level, the samples were contaminated with the stated microorganisms, and immediately after contamination, the bioburden level was calculated.

During all the days of the test, for all samples, one drop was delivered each day, 5 days every week (not actuated during weekend, but on Monday and Friday, 2 actuations instead of 1) to simulate normal patient use.

To that end, the cap of the nozzle of each bottle was removed, one drop was delivered, and the cap was screwed again. Between each dispensation, the samples were stored at the room temperature in upright position without wiping the tip.

A repeated contamination of the samples was performed at the start (D1) of the in-use testing and every two weeks during 90 days. In particular, contamination of the samples was performed on days D1, D15, D29, D43, D57 and D77, according to the above table.

The contamination of the samples was performed according to the following procedure: the cap of the nozzle of each bottle was removed, the tip of the nozzle was immersed into the contaminated solution during about 5 seconds, the tip was then removed from the contaminated solution, and the cap was screwed again. Between each dispensation, the samples were stored at the room temperature in upright position without wiping the tip.

Bioburden tests for fungi (F1-CA and F2-AB) were performed on days D29, D43, D72 and D91, corresponding to 14 days after contamination. Bioburden tests for bacteria (B1-PA and B2-SA) were performed on days D16, D30, D58 and D78, corresponding to 24h after contamination, on days D18, D32, D60 and D80, corresponding to 3 days after contamination, and also on days D29, D43, D72 and D91, corresponding to 14 days after contamination.

For performing the bioburden tests, two drops of the composition from each tested sample are delivered in a culture medium comprising tryptone salt and the number of colonies is counted to determine the number of CFUs.

Sterility testing was performed on days D18 (samples 17-32), D32 (samples 33-48), D60 (samples 49-64) and D91 (samples 65-80).

A summary is shown in the following table:

| Day | Contamination | Bioburden (fungi) | Bioburden (bacteria) | Sterility |
|---|---|---|---|---|
| D1 | ✔ | ✔ (T0) | ✔ (T0) | ✔ (T0) |
| D15 | ✔ | | | |
| D16 | | | ✔ (24h) | |
| D18 | | | ✔ (3d) | ✔ |
| D29 | ✔ | ✔ (14d) | ✔ (14d) | |
| D30 | | | ✔ (24h) | |
| D32 | | | ✔ (3d) | ✔ |
| D43 | ✔ | ✔ (14d) | ✔ (14d) | |
| D57 | ✔ | | | |
| D58 | | | ✔ (24h) | |
| D60 | | | ✔ (3d) | ✔ |
| D72 | | ✔ (14d) | ✔ (14d) | |
| D77 | ✔ | | | |
| D78 | | | ✔ (24h) | |
| D80 | | | ✔ (3d) | |
| D91 | | ✔ (14d) | ✔ (14d) | ✔ |

For sterility testing, the content of the analysed systems was sampled with a syringe after cleaning the area of drilling with alcohol. The content was put onto a filter 0.45 µm, and the filter was transferred to TSB or TRB culture medium. The cultures were incubated for 14 days at 30-35°C (TRB) and 25-25°C (TSB).

The acceptance criteria for sterility tests was absence of any growth, i.e., that the content of the device remains sterile.

In the bioburden tests for the dispensed drops, the number of colonies for each microorganism was counted to determine the colony forming units, and the log reduction was calculated at each time point.

The acceptance criteria for bioburden tests are summarized in the below table and they comply with the requirements for Ph. Eur. § 5.1.3.

| Log reduction | | | |
|---|---|---|---|
| | After 24 h | After 3 days | After 14 days |
| Bacteria | 1 | NI^{∗} | NI |
| Fungi | - | - | 1 |

| | | | |
|---|---|---|---|
| NI* - no increase | | | |

It was found that the microbial acceptance criteria, both for sterility and bioburden, were fulfilled for all time points checked along the test duration.

### Example 3 Preparation of bulk aqueous ophthalmic solution comprising dorzolamide and timolol

A solution was prepared with the ingredients listed in the following table:

| **Substance** | **% w/v (g in 100 ml)** |
|---|---|
| Dorzolamide (as dorzolamide HCI) | 2.000 (2.226) |
| Timolol (as timolol maleate) | 0.500 (0.683) |
| Hydroxyethylcellulose (grade 1,500-2,500 mPa.s) | 0.475 |
| Mannitol | 1.60 |
| Sodium citrate | 0.294 |
| NaOH/HCI | q.s. pH=5.0-6.0 |
| Purified water | q.s. |

All the ingredients were dissolved in purified water, and the pH of the solution was finally adjusted with NaOH and/or HCI.

The viscosity of the composition was 70-160 mPa·s (measured at 20°C) using a Brookfield viscosimeter.

### Example 4 Preparation a multidose ophthalmic composition comprising dorzolamide and timolol suitable for 3 months usage

The solution prepared in Example 3 was sterilized by filtration through a 0.22 µm filter. 14 ml of the sterile solution were introduced in a sterile white LDPE bottle (15 ml), having a Novelia^{®} nozzle with a tamper-proof HDPE screw cap, comprising a silver agent (Bactiglas^{®}) on the tip of the nozzle and on the inner part of the cap contacting such tip.

The multidose dispensing device comprising the dorzolamide-timolol composition was subjected to a microbiological test to assess microbiological compliance during use for a period of 91 days, following an analogous procedure as disclosed in Example 2.

It was confirmed that the microbial acceptance criteria, both for sterility and bioburden, were fulfilled for all time points checked along the test duration.

### Example 5 Preparation of bulk aqueous ophthalmic solution comprising dorzolamide and brimonidine

A solution was prepared with the ingredients listed in the following table:

| **Substance** | **% w/v (g in 100 ml)** |
|---|---|
| Dorzolamide (as dorzolamide HCI) | 2.000 (2.226) |
| Brimonidine tartrate | 0.200 |
| Polyvinylpyrrolidone | 6.000 |
| Mannitol | 1.600 |
| Sodium citrate | 0.294 |
| NaOH/HCI | q.s. pH=5.0-6.0 |
| Purified water | q.s. |

All the ingredients were dissolved in purified water, and the pH of the solution was finally adjusted with NaOH and/or HCI.

The viscosity of the composition was 75-150 mPa·s measured at 20°C using a Brookfield viscosimeter.

### Example 6 Preparation a multidose ophthalmic composition comprising dorzolamide and brimonidine suitable for 3 months usage

The solution prepared in Example 5 was sterilized by filtration through a 0.22 µm filter. 14 ml of the sterile solution were introduced in a sterile white LDPE bottle (15 ml), having a Novelia^{®} nozzle with a tamper-proof HDPE screw cap, comprising a silver agent (Bactiglas^{®}) on the tip of the nozzle and on the inner part of the cap contacting such tip.

The multidose dispensing device comprising the dorzolamide-brimonidine composition was subjected to a microbiological test to assess microbiological compliance during use for a period of 91 days, following an analogous procedure as disclosed in Example 2.

It was confirmed that the microbial acceptance criteria, both for sterility and bioburden, were fulfilled for all time points checked along the test duration.

### Example 7 Preparation of bulk aqueous ophthalmic solution comprising dorzolamide and brimonidine

A solution was prepared with the ingredients listed in the following table:

| **Substance** | **% w/v (g in 100 ml)** |
|---|---|
| Dorzolamide (as dorzolamide HCI) | 2.000 (2.230) |
| Brimonidine tartrate | 0.200 |
| Hydroxyethylcellulose | 0.525 |
| Mannitol | 2.300 |
| Sodium citrate | 0.294 |
| NaOH/HCI | q.s. pH=5.0-6.0 |
| Purified water | q.s. |

All the ingredients were dissolved in purified water, and the pH of the solution was finally adjusted with NaOH and/or HCI.

The viscosity of the composition was 75-150 mPa·s measured at 20°C using a Brookfield viscosimeter.

### Example 8 Preparation a multidose ophthalmic composition comprising dorzolamide and brimonidine suitable for 3 months usage

The solution prepared in Example 7 was sterilized by filtration through a 0.22 µm filter. 14 ml of the sterile solution were introduced in a sterile white LDPE bottle (15 ml), having a Novelia^{®} nozzle with a tamper-proof HDPE screw cap, comprising a silver agent (Bactiglas^{®}) on the tip of the nozzle and on the inner part of the cap contacting such tip.

The multidose dispensing device comprising the dorzolamide-brimonidine composition was subjected to a microbiological test to assess microbiological compliance during use for a period of 91 days, following an analogous procedure as disclosed in Example 2.

It was confirmed that the microbial acceptance criteria, both for sterility and bioburden, were fulfilled for all time points checked along the test duration.

### Example 9 Preparation of bulk aqueous ophthalmic solution comprising dorzolamide and brimonidine

A solution was prepared with the ingredients listed in the following table:

| **Substance** | **% w/v (g in 100 ml)** |
|---|---|
| Dorzolamide (as dorzolamide HCI) | 2.000 (2.230) |
| Brimonidine tartrate | 0.200 |
| Sodium carboxymethylcellulose | 1.300 |
| Mannitol | 2.000 |
| Sodium citrate | 0.294 |
| NaOH/HCI | q.s. pH=5.0-6.0 |
| Purified water | q.s. |

All the ingredients were dissolved in purified water, and the pH of the solution was finally adjusted with NaOH and/or HCI.

The viscosity of the composition was 75-150 mPa·s measured at 20°C using a Brookfield viscosimeter.

### Example 10 Preparation a multidose ophthalmic composition comprising dorzolamide and brimonidine suitable for 3 months usage

The solution prepared in Example 9 was sterilized by filtration through a 0.22 µm filter. 14 ml of the sterile solution were introduced in a sterile white LDPE bottle (15 ml), having a Novelia^{®} nozzle with a tamper-proof HDPE screw cap, comprising a silver agent (Bactiglas^{®}) on the tip of the nozzle and on the inner part of the cap contacting such tip.

The multidose dispensing device comprising the dorzolamide-brimonidine composition was subjected to a microbiological test to assess microbiological compliance during use for a period of 91 days, following an analogous procedure as disclosed in Example 2.

It was confirmed that all microbial acceptance criteria, both for sterility and bioburden, were fulfilled for all time points checked along the test duration.

## Claims

1. A multi-dose dispensing device filled with a preservative-free ophthalmic composition comprising a carbonic anhydrase inhibitor, wherein:
- the ophthalmic composition is an aqueous composition having a viscosity comprised between 50 and 200 mPa·s;
- the multi-dose dispensing device comprises a container where the ophthalmic composition is housed and a nozzle, said nozzle comprising means for preventing microbial contamination;
**characterized in that** the composition remains microbiologically acceptable during use for a period of at least 90 days after the first opening of the container as assessed in a microbiological test comprising repeated microbial challenge with four microorganisms, wherein said four microorganisms consist of one gram-negative bacterium, one gram-positive bacterium, one fungus of the *Aspergillus* genus and *Candida albicans.*

2. The multi-dose dispensing device according to claim 1, **characterized in that** the gram-negative bacterium is *Pseudomonas aeruginosa.*

3. The multi-dose dispensing device according to claim 1 or 2, **characterized in that** the gram-positive bacterium is *Staphylococcus aureus.*

4. The multi-dose dispensing device according to any one of claims 1 to 3, **characterized in that** the fungus of the *Aspergillus* genus is *Aspergillus brasiliensis.*

5. The multi-dose dispensing device according to any one of claims 1 to 4, **characterized in that** the microbiological challenge is performed suspensions of each microorganism containing about 10⁶ colony forming units (CFUs) of each germ.

6. The multi-dose dispensing device according to any one of claims 1 to 5, **characterized in that** the duration of the microbiological test is comprised between 90 and 100 days.

7. The multi-dose dispensing device according to any one of claims 1 to 6, **characterized in that** the microbial challenge is repeated at least 4 times during the testing period, preferably at least 5 times, more preferably at least 6 times, and more preferably 6 times.

8. The multi-dose dispensing device according to any one of claims 1 to 7, **characterized in that** during the testing period one drop of the composition is normally dispensed every day.

9. The multi-dose dispensing device according to any one of claims 1 to 8, **characterized in that** the means for preventing microbiological contamination consist in a closure system fitted in the nozzle.

10. The multi-dose dispensing device according to claim 9, **characterized in that** the closure system is selected from a pump system and a non-return valve combined with a filter for the air returning to the container.

11. The multi-dose dispensing device according to any one of claims 1 to 10, **characterized in that** it comprises a silver releasing antibacterial agent.

12. The multi-dose dispensing device according to any one of claims 1 to 11, **characterized in that** the carbonic anhydrase inhibitor is selected from dorzolamide, brinzolamide, and pharmaceutically acceptable salts thereof.

13. The multi-dose dispensing device according to claim 12, **characterized in that** the carbonic anhydrase inhibitor is dorzolamide or a pharmaceutically acceptable salt thereof, preferably dorzolamide hydrochloride, in an amount ranging from 0.75% to 4.0% (w/v), preferably from 1.0% to 3.0% (w/v), more preferably from 1.5% to 2.5% (w/v), still more preferably from 1.8% to 2.2% (w/v), and still more preferably the amount is about 2.0% (w/v), expressed as concentration of dorzolamide.

14. The multi-dose dispensing device according to any one of claims 1 to 13, **characterized in that** the ophthalmic composition also comprises a second anti-glaucoma active ingredient, which is preferably selected from a beta-blocker and an alpha-2 adrenergic receptor agonist.

15. The multi-dose dispensing device according to claim 14, **characterized in that** the second anti-glaucoma active ingredient is selected from timolol, or a pharmaceutically acceptable salt thereof, and brimonidine, or a pharmaceutically acceptable salt thereof.
